# EUROPEAN PATENT APPLICATION

(11) **EP 3 910 071 A1**
(43) Date of publication of application: **17.11.2021**
(21) Application number: 20738319.1
(22) Date of filing: 08.01.2020
(51) Int. Cl.: C12Q 1/6883, C12Q 1/689, C12Q 1/6886, C12Q 1/6851, A23L 33/135, A61K 9/00, A61K 35/74

(54) **NANOVESICLES DERIVED FROM BACTERIA OF GENUS RHODOCOCCUS, AND USE THEREOF**

(30) Priority: 09.01.2019 KR 20190002609; 07.01.2020 KR 20200002274
(71) Applicant: MD Healthcare Inc., Seoul 03923 (KR)
(72) Inventor: KIM, Yoon-Keun, Paju-Si, Gyeonggi-do 10908 (KR)
(74) Representative: Dehns
(86) International application number: PCT/KR2020/000342
(87) International publication number: WO 2020/145663

(57) **Abstract**

The present invention relates to vesicles derived from bacteria of the genus *Rhodococcus* and a use thereof. The inventors experimentally confirmed that the vesicles significantly decreased in a clinical sample obtained from a patient with lung cancer, pancreatic cancer, bile duct cancer, breast cancer, bladder cancer, lymphoma, diabetes, stroke, myocardial infarction, asthma, COPD, and dementia, compared with a normal individual, and when the vesicles isolated from the strain were administered, the secretion of inflammation mediators caused by pathogenic vesicles such as E. coli-derived vesicles was considerably inhibited, and vesicles derived from bacteria of the genus *Rhodococcus* significantly inhibit cranial nerve cell damage caused by stress hormones, and therefore, the vesicles derived from bacteria of the genus *Rhodococcus* according to the present invention may be effectively used to develop a method of diagnosing lung cancer, pancreatic cancer, bile duct cancer, breast cancer, bladder cancer, lymphoma, diabetes, stroke, myocardial infarction, asthma, COPD, and/or dementia, and a composition for preventing, alleviating, or treating a malignant disease, diabetes, stroke, a cardiovascular disease, an inflammatory pulmonary disease, and/or a cranial nerve disease.

## Description

### [Technical Field]

The present invention relates to nanovesicles derived from bacteria of the genus *Rhodococcus* and a use thereof, and more particularly, to a method of diagnosing a malignant disease such as lung cancer, pancreatic cancer, bile duct cancer, breast cancer, bladder cancer, lymphoma or the like, diabetes, stroke, a cardiovascular disease such as myocardial infarction or the like, an inflammatory pulmonary disease such as asthma, chronic obstructive pulmonary disease (COPD) or the like, or dementia using nanovesicles derived from bacteria of the genus *Rhodococcus,* and a composition for preventing, alleviating or treating a malignant disease, diabetes, stroke, a cardiovascular disease, an inflammatory pulmonary disease or a cranial nerve disease, which comprises the vesicles.

This application claims priority to and the benefit of Korean Patent Application No. 10-2019-0002609, filed on January 9, 2019 and Korean Patent Application No. 10-2020-0002274, filed on January 7, 2020, and all the contents disclosed in the specification and drawings of the applications are incorporated in this application.

### [Background Art]

Since the beginning of the 21st century, acute infectious diseases recognized as epidemic diseases in the past have become less important, whereas chronic inflammatory diseases accompanied by immune dysfunction caused by disharmony between humans and microbiomes have changed disease patterns as main diseases that determine the quality of life and the human lifespan. Cancer, cardiovascular diseases, chronic inflammatory diseases, metabolic diseases and neuro-psychiatric diseases, which are intractable chronic diseases in the 21st century, are major diseases that determine human lifetimes and the quality of life, and become a big challenge to public health.

It is known that the number of microorganisms coexisting in the human body has reached 100 trillion, which is 10 times more than the number of human cells, and the number of microorganism genes is more than 100 times the number of human genes. A microbiota or microbiome refers to a microbial community including bacteria, archaea and eukarya present in a given habitat.

Bacteria coexisting in our body and bacteria present in the ambient environment secrete nanometer-sized vesicles in order to exchange information on genes, low molecular compounds, proteins, and the like with other cells. The mucosa forms a physical defense membrane through which particles having a size of 200 nanometers (nm) or more cannot pass, so that bacteria coexisting in the mucosa cannot pass through the mucosa, but vesicles derived from bacteria have a size of 100 nanometers or less and are absorbed into our bodies after relatively freely passing through epithelial cells via the mucosa. Bacteria-derived vesicles that are locally secreted from bacteria are absorbed via epithelial cells of the mucous membrane to thereby induce a local inflammatory response, and the vesicles having passed through the epithelial cells are systematically absorbed via lymphatic vessels and thereby distributed in respective organs, and immune and inflammatory responses are regulated in the organs in which the vesicles are distributed. For example, vesicles derived from pathogenic gram-negative bacteria such as *Escherichia coli* locally cause colitis, and promote a systemic inflammatory response, and blood coagulation through a vascular endothelial inflammatory response when absorbed into blood vessels, and cause insulin resistance and diabetes when absorbed into insulin-acting muscle cells and the like. On the other hand, vesicles derived from beneficial bacteria may control a disease by controlling immune dysfunction and metabolic dysfunction caused by pathogenic vesicles.

As immune responses to factors such as bacteria-derived vesicles, Th17 immune responses characterized by the secretion of the interleukin (hereinafter, IL)-17 cytokine occur, and IL-6 is secreted from epithelial cells, immune cells, and the like when exposed to pathogenic bacteria-derived vesicles, thereby inducing Th17 immune responses. Inflammation caused by the Th17 immune response is characterized by neutrophil infiltration, and during the process by which inflammation occurs, tumor necrosis factor-alpha (hereinafter, TNF-α) secreted from inflammatory cells such as neutrocyte and macrophages plays an important role.

Chronic inflammation is closely related to carcinogenesis. Particularly, IL-6 and TNF-α, which are secreted from inflammatory cells such as neutrophils, promote the generation of cancer cells and the infiltration of cancer cells into peripheral tissue, inducing carcinogenesis.

A brain-derived neurotrophic factor (BDNF) is a protein in the brain generated by BDNF gene, and one of a group of neurotrophic factors, as a part of the growth factors. The factor is associated with a basic nerve growth factor, and it is known that the expression of the factor is reduced in depression, dementia, Alzheimer's disease, autism, and the like.

Meanwhile, bacteria of the genus *Rhodococcus* are aerophilic gram-positive bacteria that live widely in environments such as soil or water, and bacteria having a function of converting a harmful environmental contaminant into a non-harmful substance. Among the bacteria, *Rhodococcus equi* causes a lung disease in an immune-deficient animal due to inhalation thereof, and *Rhodococcus faciens* is known as a bacterium causing a disease in a plant such as tobacco leaves. However, to date, the fact that the bacteria of the genus *Rhodococcus* secrete vesicles extracellularly has not been reported, and particularly, no cases of application thereof in diagnosis and treatment of an intractable disease such as cancer or a cardiovascular-brain disease have been reported.

Accordingly, in the present invention, it was confirmed that vesicles derived from bacteria of the genus *Rhodococcus* significantly decrease in clinical samples of lung cancer, pancreatic cancer, bile duct cancer, breast cancer, bladder cancer, lymphoma, diabetes, stroke, myocardial infarction, asthma, chronic obstructive pulmonary disease (COPD), and dementia patients, compared to a normal individual, thereby diagnosing the diseases. In addition, as a result of isolating vesicles from *Rhodococcus equi* belonging to the genus *Rhodococcus* and analyzing the characteristic thereof, it was confirmed that they can be used as a composition for preventing, alleviating, or treating a malignant disease, diabetes, stroke, a cardiovascular disease, an inflammatory pulmonary disease, or a cranial nerve disease.

### [Disclosure]

### [Technical Problem]

As a result of conducting earnest research to solve the above conventional problems, the inventors confirmed that a content of vesicles derived from bacteria of the genus *Rhodococcus* is significantly decreased in a sample derived from a patient with a malignant disease such as lung cancer, pancreatic cancer, bile duct cancer, breast cancer, bladder cancer, lymphoma or the like, diabetes, stroke, a cardiovascular disease such as myocardial infarction or the like, an inflammatory pulmonary disease such as asthma, COPD or the like, and dementia, compared with a normal individual, through metagenomic analysis. In addition, when macrophages were treated with vesicles isolated from *Rhodococcus equi* belonging to the genus *Rhodococcus,* it was confirmed that the secretion of IL-6 and TNF-alpha, which are inflammation mediators, caused by pathogenic vesicles was remarkably inhibited, and BDNF expression inhibiting cranial nerve cell damage caused by a stress hormone significantly increased, and therefore, based on this, the present invention was completed.

Thus, an object of the present invention is to provide a method of providing information for diagnosis of lung cancer, pancreatic cancer, bile duct cancer, breast cancer, bladder cancer, lymphoma, diabetes, stroke, myocardial infarction, asthma, COPD, or dementia.

Further, another object of the present invention is to provide a composition for preventing, alleviating, or treating one or more diseases selected from the group consisting of a malignant disease, diabetes, stroke, a cardiovascular disease, an inflammatory pulmonary disease, and a cranial nerve disease, comprising vesicles derived from bacteria of the genus *Rhodococcus* as an active ingredient.

However, a technical problem to be achieved by the present invention is not limited to the aforementioned problems, and the other problems that are not mentioned may be clearly understood by a person skilled in the art from the following description.

### [Technical Solution]

To achieve the object of the present invention as described above, the present invention provides a method of providing information for diagnosing lung cancer, pancreatic cancer, bile duct cancer, breast cancer, bladder cancer, lymphoma, diabetes, stroke, myocardial infarction, asthma, chronic obstructive pulmonary disease (COPD), or dementia, the method comprising the following steps:
(a) extracting DNAs from vesicles isolated from samples of a normal individual and a subj ect;
(b) performing PCR (Polymerase Chain Reaction) on the extracted DNA using a pair of primers prepared based on a gene sequence present in 16S rDNA; and
(c) classifying a case in which a content of vesicles derived from bacteria of the genus *Rhodococcus* is lower than that of the normal individual sample, as lung cancer, pancreatic cancer, bile duct cancer, breast cancer, bladder cancer, lymphoma, diabetes, stroke, myocardial infarction, asthma, COPD, or dementia, through quantitative analysis of the PCR product.

In addition, the present invention provides a method of diagnosing lung cancer, pancreatic cancer, bile duct cancer, breast cancer, bladder cancer, lymphoma, diabetes, stroke, myocardial infarction, asthma, chronic obstructive pulmonary disease (COPD), or dementia, the method comprising the following steps:
(a) extracting DNAs from vesicles isolated from samples of a normal individual and a subj ect;
(b) performing PCR on the extracted DNA using a pair of primers prepared based on a gene sequence present in 16S rDNA to obtain each PCR product; and
(c) determining a case in which a content of vesicles derived from bacteria of the genus *Rhodococcus* is lower than that of the normal individual sample, as lung cancer, pancreatic cancer, bile duct cancer, breast cancer, bladder cancer, lymphoma, diabetes, stroke, myocardial infarction, asthma, COPD, or dementia, through quantitative analysis of the PCR product.

As an embodiment of the present invention, the sample in Step (a) is not limited, but may be blood, urine, stool, saliva or nasal mucosa, and preferably blood or urine.

As another embodiment of the present invention, the primer pair in Step (b) may be primers of SEQ ID Nos. 1 and 2.

Further, the present invention provides a pharmaceutical composition for preventing or treating one or more diseases selected from the group consisting of a malignant disease, diabetes, stroke, a cardiovascular disease, an inflammatory pulmonary disease, and a cranial nerve disease, comprising vesicles derived from bacteria of the genus *Rhodococcus* as an active ingredient.

Further, the present invention provides a food composition for preventing or alleviating one or more diseases selected from the group consisting of a malignant disease, diabetes, stroke, a cardiovascular disease, an inflammatory pulmonary disease, and a cranial nerve disease, comprising vesicles derived from bacteria of the genus *Rhodococcus* as an active ingredient.

Further, the present invention provides an inhalant composition for preventing or treating one or more diseases selected from the group consisting of a malignant disease, diabetes, stroke, a cardiovascular disease, an inflammatory pulmonary disease, and a cranial nerve disease, comprising vesicles derived from bacteria of the genus *Rhodococcus* as an active ingredient.

Further, the present invention provides a method of preventing or treating one or more diseases selected from the group consisting of a malignant disease, diabetes, stroke, a cardiovascular disease, an inflammatory pulmonary disease, and a cranial nerve disease, the method comprising a step of administering a pharmaceutical composition comprising vesicles derived from bacteria of the genus *Rhodococcus* as an active ingredient to a subject.

Further, the present invention provides a use of a pharmaceutical composition comprising vesicles derived from bacteria of the genus *Rhodococcus* as an active ingredient for preventing or treating one or more diseases selected from the group consisting of a malignant disease, diabetes, stroke, a cardiovascular disease, an inflammatory pulmonary disease, and a cranial nerve disease.

Further, the present invention provides a use of vesicles derived from bacteria of the genus *Rhodococcus* for producing a drug used in treating one or more diseases selected from the group consisting of a malignant disease, diabetes, stroke, a cardiovascular disease, an inflammatory pulmonary disease, and a cranial nerve disease.

As an embodiment of the present invention, the malignant disease may be one or more selected from the group consisting of lung cancer, pancreatic cancer, bile duct cancer, breast cancer, bladder cancer, and lymphoma.

As another embodiment of the present invention, the cardiovascular disease may be one or more selected from the group consisting of myocardial infarction, hypertension, ischemic heart disease, coronary artery disease, angina, atherosclerosis (arteriosclerosis), and arrhythmia.

As another embodiment of the present invention, the inflammatory pulmonary disease may be one or more selected from the group consisting of asthma, COPD, acute lung injury, empyema, lung abscesses, pneumonia, pulmonary tuberculosis, and bronchitis.

As another embodiment of the present invention, the cranial nerve disease may be one or more selected from the group consisting of depression, obsessive-compulsive disorder, schizophrenia, dementia, Alzheimer's disease, epilepsy, autism, and Parkinson's disease.

Further, the present invention provides a cosmetic composition for preventing or alleviating an inflammatory skin disease, comprising vesicles derived from bacteria of the genus *Rhodococcus* as an active ingredient.

As an embodiment of the present invention, the inflammatory skin disease may be one or more selected from the group consisting of atopic dermatitis, acne, hair loss, and psoriasis.

As an embodiment of the present invention, the vesicles may have an average diameter of 10 to 200 nm.

As another embodiment of the present invention, the vesicles may be secreted naturally or artificially from bacteria of the genus *Rhodococcus.*

As another embodiment of the present invention, the vesicles derived from bacteria of the genus *Rhodococcus* may be secreted from *Rhodococcus equi.*

### [Advantageous Effects]

The inventors confirmed that intestinal bacteria are not absorbed into the body through epithelial cells, but bacteria-derived vesicles are absorbed, systemically distributed and then excreted out of the body through the kidneys, liver and lungs, and by metagenomic analysis for vesicles derived from bacteria present in patients' blood, also confirmed that vesicles derived from bacteria of the genus *Rhodococcus,* which are present in blood of patients with lung cancer, pancreatic cancer, bile duct cancer, breast cancer, bladder cancer, lymphoma, diabetes, stroke, myocardial infarction, asthma, COPD, and dementia significantly decrease, compared with a normal individual. In addition, when inflammatory cells were administered in vitro with vesicles in vitro cultured and isolated from *Rhodococcus equi* which is one bacterial species of the genus *Rhodococcus,* it was observed that the secretion of inflammation mediators caused by an inflammatory factor is significantly inhibited. In addition, as it is confirmed that the BDNF expression of nerve cells, which is inhibited by a stress hormone, is significantly recovered by the vesicles, the vesicles derived from bacteria of the genus *Rhodococcus* according to the present invention are expected to be effectively used in a method of diagnosing lung cancer, pancreatic cancer, bile duct cancer, breast cancer, bladder cancer, lymphoma, diabetes, stroke, myocardial infarction, asthma, COPD, or dementia, and a food, inhalant or pharmaceutical composition for preventing or treating a malignant disease, diabetes, stroke, a cardiovascular disease, an inflammatory pulmonary disease, and a cranial nerve disease.

### [Description of Drawings]

FIG. 1A is a series of photographs capturing distribution patterns of bacteria and bacteria-derived vesicles (EV) by time after the bacteria and the vesicles derived from bacteria were orally administered to mice, and FIG. 1B is a result of evaluating the in vivo distribution patterns of the bacteria and the vesicles by harvesting blood, kidneys, liver, and various organs at 12 hours after orally administering the bacteria and the vesicles.
FIGS. 2A and 2B are results obtained by comparing the distribution of vesicles derived from bacteria of the genus *Rhodococcus* after metagenomic analysis is performed on vesicles derived from bacteria present in a lung cancer patient and a normal individual, in which FIG. 2A is the result using a blood sample, and FIG. 2B is the result using a urine sample.
FIG. 3 is a result of comparing the distributions of vesicles derived from bacteria of the genus *Rhodococcus* after metagenomic analysis of bacteria-derived vesicles present in the blood of pancreatic cancer patients and a normal individual.
FIG. 4 is a result of comparing the distributions of vesicles derived from bacteria of the genus *Rhodococcus* after metagenomic analysis of bacteria-derived vesicles present in the blood of bile duct cancer patients and a normal individual.
FIG. 5 is a result of comparing the distributions of vesicles derived from bacteria of the genus *Rhodococcus* after metagenomic analysis of bacteria-derived vesicles present in the blood of breast cancer patients and a normal individual.
FIG. 6 is a result of comparing the distributions of vesicles derived from bacteria of the genus *Rhodococcus* after metagenomic analysis of bacteria-derived vesicles present in the blood of bladder cancer patients and a normal individual.
FIG. 7 is a result of comparing the distributions of vesicles derived from bacteria of the genus *Rhodococcus* after metagenomic analysis of bacteria-derived vesicles present in the blood of lymphoma patients and a normal individual.
FIG. 8 is a result of comparing the distributions of vesicles derived from bacteria of the genus *Rhodococcus* after metagenomic analysis of bacteria-derived vesicles present in the blood of diabetes patients and a normal individual.
FIG. 9 is a result of comparing the distributions of vesicles derived from bacteria of the genus *Rhodococcus* after metagenomic analysis of bacteria-derived vesicles present in the blood of stroke patients and a normal individual.
FIG. 10 is a result of comparing the distributions of vesicles derived from bacteria of the genus *Rhodococcus* after metagenomic analysis of bacteria-derived vesicles present in the blood of myocardial infarction patients and a normal individual.
FIG. 11 is a result of comparing the distributions of vesicles derived from bacteria of the genus *Rhodococcus* after metagenomic analysis of bacteria-derived vesicles present in the blood of asthma patients and a normal individual.
FIG. 12 is a result of comparing the distributions of vesicles derived from bacteria of the genus *Rhodococcus* after metagenomic analysis of bacteria-derived vesicles present in the blood of COPD patients and a normal individual.
FIG. 13 is a result of comparing the distributions of vesicles derived from bacteria of the genus *Rhodococcus* after metagenomic analysis of bacteria-derived vesicles present in the blood of dementia patients and a normal individual.
FIGS. 14A and 14B are results obtained by evaluating the influence on inflammation mediator secretion by *E. coli* vesicles by pretreating vesicles derived from *Rhodococcus* bacteria before treatment of *E. coli* vesicles (*E. coli* EV), which are pathogenic vesicles, to evaluate anti-inflammatory and immunoregulatory effects of *Rhodococcus equi-derived* vesicles, in which FIG. 14A is the result of comparing the degree of IL-6 secretion, and FIG. 14B is result of comparing the degree of TNF-α secretion.
FIG. 15 shows the result of evaluating the influence on brain-derived neurotrophic factor (BDNF) expression by nerve cells by simultaneously treating nerve cells, which have been treated with an adrenocortical hormone (GC), which is a stress hormone, with *Rhodococcus* equi-derived vesicles to evaluate a nerve cell protective effect of the *Rhodococcus* equi-derived vesicles (EV: *Rhodococcus equi* extracellular vesicle).

### [Best Modes]

The present invention relates to vesicles derived from bacteria of the genus *Rhodococcus* and a use thereof.

The inventors confirmed that a content of vesicles derived from bacteria of the genus *Rhodococcus* are significantly reduced in samples of patients with lung cancer, pancreatic cancer, bile duct cancer, breast cancer, bladder cancer, lymphoma, diabetes, stroke, myocardial infarction, asthma, COPD, and dementia, compared with a normal individual, through metagenomic analysis. In addition, when macrophages were treated with vesicles isolated from *Rhodococcus equi* belonging to the genus *Rhodococcus,* it was confirmed that the secretion of IL-6 and TNF-alpha, which are inflammation mediators, caused by pathogenic vesicles was remarkably inhibited, and the BDNF expression of nerve cells, which is inhibited by a stress hormone, was significantly recovered by the vesicles, and based on this, the present invention was completed.

Thus, the present invention provides a method of providing information for diagnosing lung cancer, pancreatic cancer, bile duct cancer, breast cancer, bladder cancer, lymphoma, diabetes, stroke, myocardial infarction, asthma, COPD, or dementia, the method comprising the following steps:
(a) extracting DNAs from vesicles isolated from samples of a normal individual and a subject;
(b) performing PCR on the extracted DNA using a pair of primers prepared based on a gene sequence present in 16S rDNA to obtain each PCR product; and
(c) classifying a case in which a content of vesicles derived from bacteria of the genus *Rhodococcus* is lower than that of the normal individual sample, as lung cancer, pancreatic cancer, bile duct cancer, breast cancer, bladder cancer, lymphoma, diabetes, stroke, myocardial infarction, asthma, COPD, or dementia, through quantitative analysis of the PCR product.

The term "diagnosis" as used herein refers to determination of a condition of a disease of a patient over all aspects, in a broad sense. The contents of the determination are the disease entity, the etiology, the pathogenesis, the severity, the detailed aspects of a disease, the presence and absence of complications, the prognosis, and the like. The diagnosis in the present invention means determining whether lung cancer, pancreatic cancer, bile duct cancer, breast cancer, bladder cancer, lymphoma, diabetes, stroke, myocardial infarction, asthma, COPD, and/or dementia occur, the level of the disease, and the like.

The term "nanovesicle" or "vesicle" as used herein refers to a structure consisting of a nano-sized membrane secreted from various bacteria. Vesicles derived from gram-negative bacteria or outer membrane vesicles (OMVs) have endotoxins (lipopolysaccharides), toxic protein, and bacterial DNA and RNA, and vesicles derived from gram-positive bacteria also have peptidoglycan and lipoteichoic acid which are cell wall components of bacteria in addition to proteins and nucleic acids. In the present invention, nanovesicles or vesicles are secreted naturally from bacteria of the genus *Rhodococcus* or produced artificially, are in the form of a sphere, and have an average diameter of 10 to 200 nm.

The term "metagenome" as used herein also refers to a microbiome, and refers to a total of genomes including all viruses, bacteria, fungi, and the like in an isolated region such as soil and an animal's intestines, and is typically used as a concept of genomes explaining identification of a large number of microorganisms at one time by using a sequence analyzer in order to analyze uncultivated microorganisms. In particular, the metagenome does not refer to a genome of one species, but refers to a kind of mixed genome as a genome of all species of one environmental unit. The metagenome is, when one species is defined in the development process of omics biology, a term derived from the viewpoint of making a complete species is made by various species interacting with each other as well as one kind of functionally existing species. Technically, the metagenome is an object of a technique to identify all species in one environment and investigate interactions and metabolism by analyzing all DNAs and RNAs regardless of species using a rapid sequence analysis method.

In the present invention, the sample derived from patients is not limited, but may be blood, urine, stool, saliva or nasal mucosa, and preferably blood or urine.

In the present invention, the primer pair in Step (b) may be primers of SEQ ID Nos. 1 and 2, but is not limited thereto.

Another aspect of the present invention provides a composition for preventing or treating one or more diseases selected from the group consisting of a malignant disease, diabetes, stroke, a cardiovascular disease, an inflammatory pulmonary disease, and a cranial nerve disease, comprising vesicles derived from bacteria of the genus *Rhodococcus* as an active ingredient.

The composition includes a pharmaceutical composition and an inhalant composition.

Further, the present invention provides a method of preventing or treating one or more diseases selected from the group consisting of a malignant disease, diabetes, stroke, a cardiovascular disease, an inflammatory pulmonary disease, and a cranial nerve disease, the method comprising a step of administering a pharmaceutical composition comprising vesicles derived from bacteria of the genus *Rhodococcus* as an active ingredient to a subject.

Further, the present invention provides a use of a pharmaceutical composition comprising vesicles derived from bacteria of the genus *Rhodococcus* as an active ingredient for preventing or treating one or more diseases selected from the group consisting of a malignant disease, diabetes, stroke, a cardiovascular disease, an inflammatory pulmonary disease, and a cranial nerve disease.

Further, the present invention provides a use of vesicles derived from bacteria of the genus *Rhodococcus* for producing a drug used in treating one or more diseases selected from the group consisting of a malignant disease, diabetes, stroke, a cardiovascular disease, an inflammatory pulmonary disease, and a cranial nerve disease.

The term "prevention" as used herein refers to all actions that suppress a malignant disease, diabetes, stroke, a cardiovascular disease, an inflammatory pulmonary disease, a cranial nerve disease, or the like, or delay the onset thereof via administration of the composition according to the present invention.

The term "treatment" as used herein refers to all actions that alleviate or beneficially change symptoms of a malignant disease, diabetes, stroke, a cardiovascular disease, an inflammatory pulmonary disease, a cranial nerve disease, or the like via administration of composition according to the present invention.

The term "alleviation" used as used herein refers to all actions that at least reduce a parameter associated with a condition to be treated, for example, the degree of symptoms.

The term "subject" used herein refers to a target in need of treatment of one or more diseases selected from the group consisting of a malignant disease, diabetes, stroke, a cardiovascular disease, an inflammatory pulmonary disease, and a cranial nerve disease, and more specifically, a mammal such as a human or a non-human primate, a mouse, a dog, a cat, a horse, a cow, and the like.

The term "administration" used herein means providing a composition of the present invention to a subject by any suitable method.

The term "malignant disease" used herein refers to all diseases with extremely poor prognosis, such as cancer and sarcomas, and diseases that are difficult to treat and have fast progression, and in the present invention, the malignant disease may be one or more selected from the group consisting of lung cancer, pancreatic cancer, bile duct cancer, breast cancer, bladder cancer and lymphoma, but the present invention is not limited thereto.

The term "cardiovascular disease" used herein is a generic term for diseases occurring in the heart and the major arteries, and in the present invention, the cardiovascular disease may be one or more selected from the group consisting of myocardial infarction, hypertension, ischemic heart disease, coronary artery disease, angina, atherosclerosis (arteriosclerosis) and arrhythmia, but the present invention is not limited thereto.

The term "inflammatory pulmonary disease" used herein refers to a disease caused by an inflammatory response associated with the lungs, and in the present invention, the inflammatory pulmonary disease may be one or more selected from the group consisting of asthma, COPD, acute lung injury, empyema, lung abscesses, pneumonia, pulmonary tuberculosis and bronchitis, but the present invention is not limited thereto.

The term "cranial nerve disease" used herein is a generic term for diseases caused by problems with cranial nerve cells, and in the present invention, the cranial nerve disease may be one or more selected from the group consisting of depression, obsessive-compulsive disorder, schizophrenia, dementia, Alzheimer's disease, epilepsy, autism and Parkinson's disease, but the present invention is not limited thereto.

The vesicles may be isolated from a culturing solution comprising bacteria of the genus *Rhodococcus* by using one or more methods selected from the group consisting of centrifugation, ultra-high speed centrifugation, high pressure treatment, extrusion, sonication, cell lysis, homogenization, freezing-thawing, electroporation, mechanical decomposition, chemical treatment, filtration by a filter, gel filtration chromatography, free-flow electrophoresis, and capillary electrophoresis. Further, a process such as washing for removing impurities and concentration of obtained vesicles may be further included.

The pharmaceutical composition of the present invention may include a pharmaceutically acceptable carrier. The pharmaceutically acceptable carrier is typically used in formulation, and includes saline, sterile water, Ringer's solution, buffered saline, cyclodextrin, a dextrose solution, a maltodextrin solution, glycerol, ethanol, liposomes, and the like, but is not limited thereto, and may further include other typical additives such as an antioxidant and a buffer, if necessary. Further, the composition may be formulated into an injectable formulation, such as an aqueous solution, a suspension, and an emulsion, a pill, a capsule, a granule, or a tablet by additionally adding a diluent, a dispersant, a surfactant, a binder, a lubricant, and the like. With regard to suitable pharmaceutically acceptable carriers and formulations, the composition may be preferably formulated according to each ingredient by using the method disclosed in the Remington's literature. The pharmaceutical composition of the present invention is not particularly limited in formulation, but may be formulated into an injection, an inhalant, an external preparation for skin, an oral ingestion, or the like.

The pharmaceutical composition of the present invention may be administered orally or parenterally (e.g., intravenously, subcutaneously, intradermally) according to a desired method, and a dose may vary according to the condition and body weight of a patient, the severity of a disease, a drug formulation, an administration route, and duration, but may be suitably selected by those of ordinary skill in the art.

The pharmaceutical composition according to the present invention is administered in a pharmaceutically effective amount. In the present invention, the pharmaceutically effective amount refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields. The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

Specifically, the effective amount of the pharmaceutical composition according to the present invention may be changed according to a patient's age, sex or body weight, and may be increased or decreased depending on the route of administration, the severity of obesity, sex, a body weight, age or the like.

Another aspect of the present invention provides a food composition for preventing or alleviating one or more diseases selected from the group consisting of a malignant disease, diabetes, stroke, a cardiovascular disease, an inflammatory pulmonary disease, and a cranial nerve disease, comprising vesicles derived from bacteria of the genus *Rhodococcus* as an active ingredient.

The food composition of the present invention includes a health functional food composition. The food composition according to the present invention may be used by adding an active ingredient as is to food or may be used together with other foods or food ingredients, but may be appropriately used according to a typical method. The mixed amount of the active ingredient may be suitably determined depending on the purpose of use thereof (for prevention or alleviation). In general, when a food or beverage is prepared, the composition of the present invention is added in an amount of 15 wt% or less, preferably 10 wt% or less based on the raw materials. However, for long-term intake for the purpose of health and hygiene or for the purpose of health control, the amount may be less than the above-mentioned range.

Other ingredients are not particularly limited, except that the food composition of the present invention contains the active ingredient as an essential ingredient at the indicated ratio, and the food composition of the present invention may contain various flavorants, natural carbohydrates, and the like, like a typical beverage, as an additional ingredient. Examples of the above-described natural carbohydrate include common sugars such as monosaccharides, for example, glucose, fructose and the like; disaccharides, for example, maltose, sucrose and the like; and polysaccharides, for example, dextrin, cyclodextrin and the like, and sugar alcohols such as xylitol, sorbitol, and erythritol. As the flavorant other than those described above, a natural flavorant (thaumatin, stevia extract, for example, rebaudioside A, glycyrrhizin and the like), and a synthetic flavorant (saccharin, aspartame and the like) may be advantageously used. The proportion of the natural carbohydrate may be appropriately determined by the choice of those of ordinary skill in the art.

The food composition of the present invention may contain various nutrients, vitamins, minerals (electrolytes), flavoring agents such as synthetic flavoring agents and natural flavoring agents, colorants and fillers (cheese, chocolate, and the like), pectic acid and salts thereof, alginic acid and salts thereof, organic acids, protective colloid thickeners, pH adjusting agents, stabilizers, preservatives, glycerin, alcohols, carbonating agents used in a carbonated beverage, or the like, in addition to the additives. These ingredients may be used either alone or in combinations thereof. The ratio of these additives may also be appropriately selected by those of ordinary skill in the art.

The inhalant composition of the present invention may include not only vesicles derived from bacteria of the genus *Rhodococcus,* but also components conventionally used in inhalant compositions, for example, conventional adjuvants such as an antioxidant, a stabilizer, a solubilizer, a vitamin and a flavoring agent, and a carrier.

Another aspect of the present invention provides a cosmetic composition for preventing or alleviating an inflammatory skin disease, comprising vesicles derived from bacteria of the genus *Rhodococcus* as an active ingredient.

In the present invention, the inflammatory skin disease may be one or more selected from the group consisting of atopic dermatitis, acne, hair loss, and psoriasis, but is not limited thereto.

The cosmetic composition of the present invention may comprise ingredients conventionally used in a cosmetic composition as well as vesicles derived from bacteria of the genus *Rhodococcus,* and may comprise, for example, a conventional additive such as an antioxidant, a stabilizer, a solubilizer, a vitamin, a pigment and a flavor, and a carrier.

In addition, the composition of the present invention may also be used by mixing a conventionally used organic sunscreen as long as it does not impair a skin protection effect by reaction with the vesicles derived from bacteria of the genus *Rhodococcus,* in addition to the vesicles derived from bacteria of the genus *Rhodococcus.* The organic sunscreen may be one or more selected from the group consisting of glyceryl PABA, drometrizole trisiloxane, drometrizole, digalloyl trioleate, disodium phenyl dibenzimidazole tetrasulfonate, diethylhexyl butamidotriazone, diethylamino hydroxybenzoyl hexylbenzoate, DEA-methoxycinnamate, a Lawson/dihydroxyacetone mixture, methylenebis-benzotriazolyltetramethylbutylphenol, 4-methylbenzylidene camphor, methyl anthranilate, benzophenone-3(oxybenzone), benzophenone-4, benzophenone-8(dioxyphebenzone), butyl methoxydibenzoylmethane, bisethylhexyloxyphenol methoxyphenyl triazine, cinoxate, ethyl dihydroxypropyl PABA, octocrylene, ethylhexyldimethyl PABA, ethylhexyl methoxycinnamate, ethylhexyl salicylate, ethylhexyl triazone, isoamyl-p-methoxycinnamate, polysilicon-15 (dimethicodiethylbenzal malonate), terephthalylidene dicamphor sulfonic acid and a salt thereof, TEA-salicylate and aminobenzoic acid (PABA).

Products that can contain the cosmetic composition of the present invention include, for example, cosmetics such as an astringent, a skin toner, a nourishing toner, various types of creams, essences, packs and foundations, cleansers, face washes, soaps, treatments, and tonics. Specific formulations of the cosmetic composition of the present invention include a skin lotion, a skin softener, a skin toner, an astringent, a lotion, a milk lotion, a moisturizing lotion, a nourishing lotion, a massage cream, a nourishing cream, a moisturizing cream, a hand cream, an essence, a nourishing essence, a pack, a soap, a shampoo, a cleansing foam, a cleansing lotion, a cleansing cream, a body lotion, a body cleanser, an emulsion, a lipstick, a makeup base, a foundation, a pressed powder, a loose powder, and an eyeshadow.

In one embodiment of the present invention, as a result of orally administering bacteria and bacteria-derived vesicles to mice and observing in vivo absorption, distribution, and excretion patterns of the bacteria and the vesicles, it was confirmed that, while the bacteria were not absorbed via the intestinal mucous membrane, the bacteria-derived vesicles were absorbed within 5 minutes after administration and systemically distributed, and excreted via the kidneys, liver, and the like (see Example 1).

In another embodiment of the present invention, a bacterial metagenomic analysis was performed by using vesicles isolated from the blood or urine of normal individuals who were matched in age and sex with patients with lung cancer, pancreatic cancer, bile duct cancer, breast cancer, bladder cancer, lymphoma, diabetes, stroke, myocardial infarction, asthma, COPD, and dementia. As a result, it was confirmed that vesicles derived from bacteria of the genus *Rhodococcus* were significantly decreased in clinical samples of patients with lung cancer, pancreatic cancer, bile duct cancer, breast cancer, bladder cancer, lymphoma, diabetes, stroke, myocardial infarction, asthma, COPD, and dementia as compared to samples of normal individuals (see Examples 3 to 14).

In still another embodiment of the present invention, it was evaluated whether vesicles secreted from the cultured *Rhodococcus equi* strain exhibit immunoregulatory and anti-inflammatory effects, and as a result of evaluating the secretion of an inflammation mediator by treating macrophages with various concentrations of the *Rhodococcus* equi-derived vesicles, and then with *E*. *coli*-derived vesicles, which is an inflammatory disease-causing factor, it was confirmed that the secretion of IL-6 and TNF-α caused by E. coli-derived vesicles is efficiently inhibited by the *Rhodococcus* equi-derived vesicles (see Example 16).

In still another embodiment of the present invention, the nerve cell protective effect of vesicles derived from a *Rhodococcus equi* strain was evaluated, and as a result of evaluating brain-derived neurotrophic factor (BDNF) expression by simultaneously treating nerve cells, which have been treated with an adrenocortical hormone, which stresses nerve cells, with *Rhodococcus* equi-derived vesicles, it was confirmed that the *Rhodococcus equi-derived* vesicles effectively increase the expression of BDNF, which is a mediator protecting against nerve cell damage (see Example 17).

Hereinafter, preferred Examples for helping the understanding of the present invention will be suggested. However, the following Examples are provided only to more easily understand the present invention, and the contents of the present invention are not limited by the following Examples.

### [Examples]

### Example 1. Analysis of in vivo Absorption, Distribution, and Excretion Patterns of Intestinal Bacteria and Vesicles Derived from Bacteria

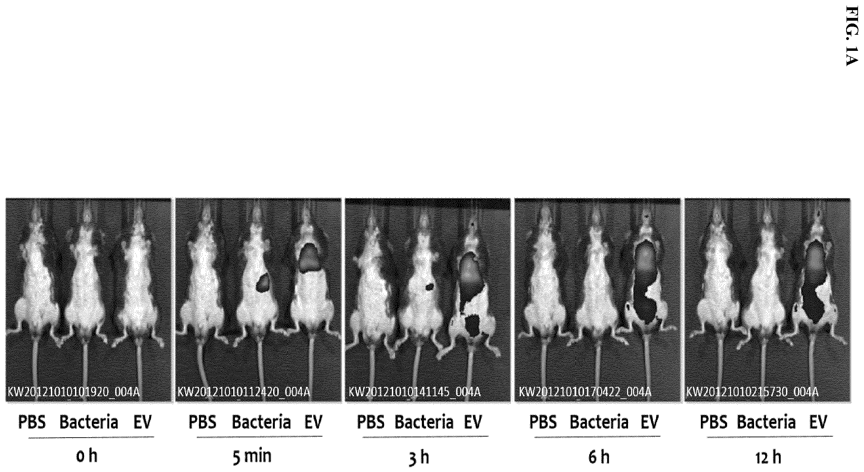

In order to evaluate whether bacteria and bacteria-derived vesicles were systemically absorbed through the mucous, an experiment was performed with the following method. First, a dose of 50 µg of each of fluorescence-labeled intestinal bacteria and the intestinal bacteria-derived vesicles was administered to the stomach of a mouse, and fluorescence was measured after 0 minute, 5 minutes, 3 hours, 6 hours, and 12 hours. As a result of observing the entire image of the mouse, as illustrated in FIG. 1A, the bacteria were not systemically absorbed, but the vesicles derived from bacteria were systemically absorbed 5 minutes after administration, and fluorescence was strongly observed in the bladder 3 hours after administration, so that it could be seen that the vesicles were excreted to the urinary tract. Further, it could be seen that the vesicles were present in the body until 12 hours after administration (see FIG. 1A).

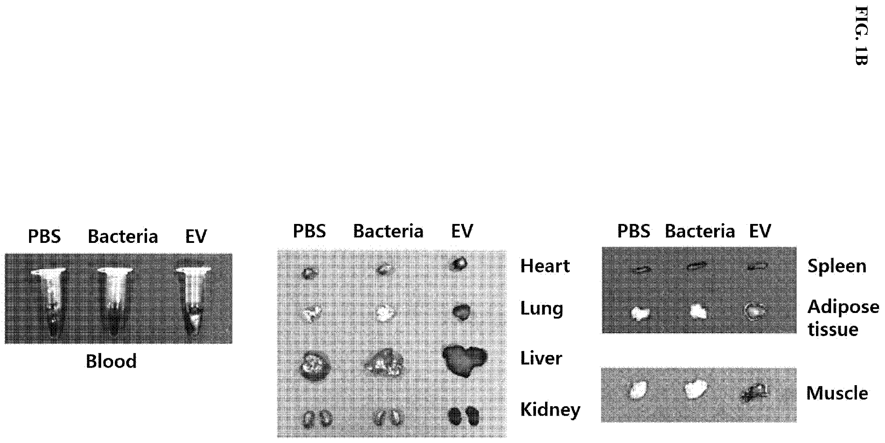

In addition, in order to evaluate the pattern in which the intestinal bacteria and the vesicles derived from the intestinal bacteria infiltrated into various organs after they were systemically absorbed, 50 µg of bacteria and vesicles derived from bacteria labeled with fluorescence were administered in the same manner as described above, and then the urine, heart, lungs, liver, kidneys, spleen, fat, and muscle were collected 12 hours after administration. As a result of observing fluorescence in the collected tissues, as illustrated in FIG. 1B, it could be seen that the vesicles derived from bacteria were distributed in the urine, heart, lungs, liver, spleen, fat, muscle, and kidneys but the bacteria were not absorbed (see FIG. 1B).

### Example 2. Metagenomic Analysis of Vesicles Derived from Bacteria in Clinical Sample

After blood or urine was first put into a 10-ml tube and suspended matter was allowed to settle by a centrifuge (3,500 × g, 10 min, 4°C), only the supernatant was transferred to a new 10-ml tube. After bacteria and impurities were removed by using a 0.22-µm filter, they were transferred to a Centriprep tube (centrifugal filters 50 kD) and centrifuged at 1,500 × g and 4°C for 15 minutes, materials smaller than 50 kD were discarded, and the residue was concentrated to 10 ml. After bacteria and impurities were removed once again by using a 0.22-µm filter, the supernatant was discarded by using a ultra-high speed centrifugation at 150,000 × g and 4°C for 3 hours with a Type 90Ti rotor, and an aggregated pellet was dissolved in physiological saline (PBS).

Internal DNA was extracted out of the lipid by boiling 100 µl of the vesicles isolated by the above method at 100°C, and then cooled on ice for 5 minutes. And then, in order to remove the remaining suspended matter, the DNA was centrifuged at 10,000 × g and 4°C for 30 minutes, and only the supernatant was collected. And, the amount of DNA was quantified by using Nanodrop.

Thereafter, in order to confirm whether the DNA derived from bacteria was present in the extracted DNA, PCR was performed with 16s rDNA primers shown in the following Table 1 and it was confirmed that genes derived from bacteria were present in the extracted genes.

**[Table 1]**

| primer | | Sequence | SEQ ID No. |
|---|---|---|---|
| 16S rDNA | 16S_V3_F | 5'- TCGTCGGCAGCGTCAGATGTGTATAAGA GACAGCCTACGGGNGGCWGCAG-3' | 1 |
| | 16S_V4_R | 5'- GTCTCGTGGGCTCGGAGATGTGTATAAG AGACAGGACTACHVGGGTATCTAATCC | 2 |

The DNA extracted by the above method was amplified using the 16S rDNA primers, and then sequencing was performed (Illumina MiSeq sequencer), the results were output as a standard flowgram format (SFF) file, the SFF file was converted into a sequence file (.fasta) and a nucleotide quality score file using GS FLX software (v2.9), and then the reliability estimation for the reads was confirmed, and a portion in which the window (20 bps) average base call accuracy was less than 99% (Phred score<20) was removed. For the OTU (operational taxonomy unit) analysis, clustering was performed according to sequence similarity by using UCLUST and USEARCH, the genus, family, order, class, and phylum were clustered based on 94%, 90%, 85%, 80%, and 75% sequence similarity, respectively, classification was performed at the phylum, class, order, family, and genus levels of each OUT, and bacteria having a sequence similarity of 97% or more at the genus level were profiled by using the 16S RNA sequence database (108,453 sequences) of BLASTN and GreenGenes (QIIME).

### Example 3. Metagenomic analysis of bacteria-derived vesicles in blood and urine of patient with lung cancer

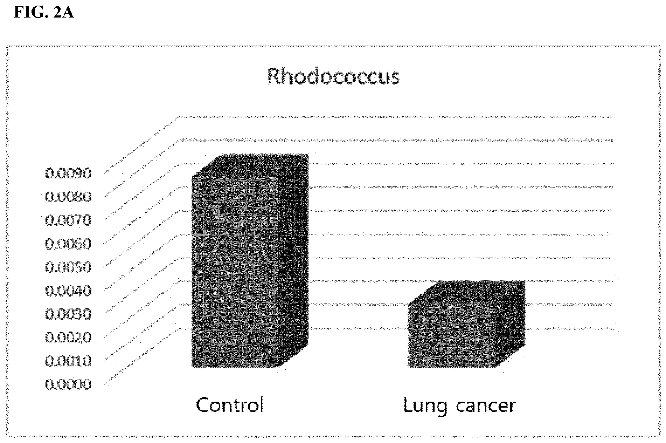

After a metagenomic analysis was performed using the method of Example 2 on the blood from 126 patients with lung cancer, and 198 normal individuals who were matched in age and sex by extracting genes from vesicles present in the blood, the distribution of vesicles derived from bacteria of the genus *Rhodococcus* was evaluated. As a result, it was confirmed that vesicles derived from bacteria of the genus *Rhodococcus* were significantly decreased in the blood from the patients with lung cancer as compared to the blood from the normal individuals (see Table 2 and FIG. 2A).

**[Table 2]**

| Blood | Control | | Lung cancer | | t-test | |
|---|---|---|---|---|---|---|
| Taxon | Mean | SD | Mean | SD | p-value | Ratio |
| *Rodocococcus* | 0.0081 | 0.0364 | 0.0027 | 0.0102 | 0.02 | 0.33 |

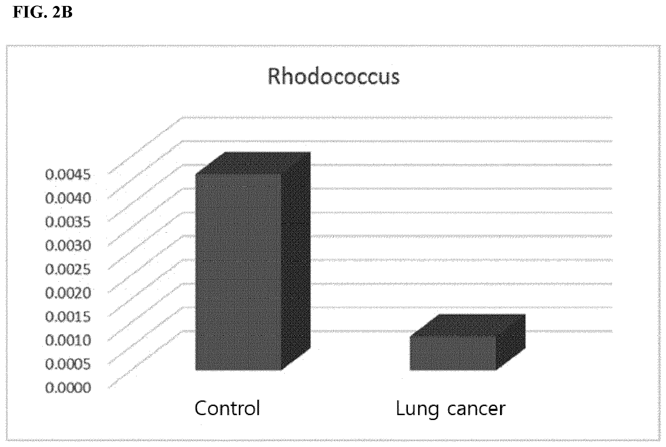

After a metagenomic analysis was performed using the method of Example 2 on the urine from 66 patients with lung cancer, and 78 normal individuals who were matched in age and sex by extracting genes from vesicles present in the urine, the distribution of vesicles derived from bacteria of the genus *Rhodococcus* was evaluated. As a result, it was confirmed that vesicles derived from bacteria of the genus *Rhodococcus* were significantly decreased in the urine from the patients with lung cancer as compared to the urine from the normal individuals (see Table 3 and FIG. 2B).

**[Table 3]**

| Urine | Control | | Lung cancer | | t-test | |
|---|---|---|---|---|---|---|
| Taxon | Mean | SD | Mean | SD | p-value | Ratio |
| *Rodocococcus* | 0.0041 | 0.0105 | 0.0007 | 0.0027 | 0.008 | 0.17 |

### Example 4. Metagenomic analysis of bacteria-derived vesicles in blood of patient with pancreatic cancer

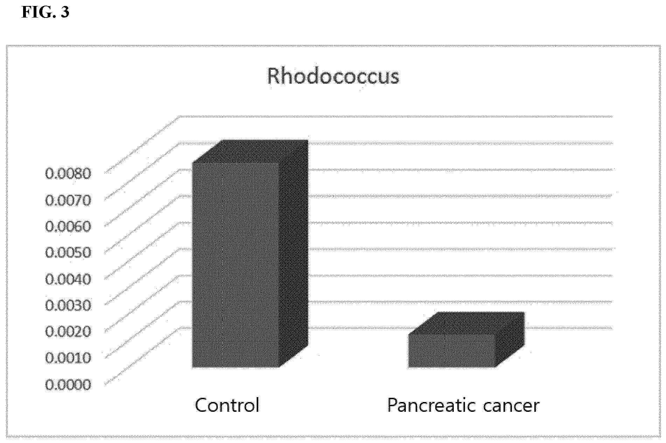

After a metagenomic analysis was performed using the method of Example 2 on the blood from 176 patients with pancreatic cancer, and 271 normal individuals who were matched in age and sex by extracting genes from vesicles present in the blood, the distribution of vesicles derived from bacteria of the genus *Rhodococcus* was evaluated. As a result, it was confirmed that vesicles derived from bacteria of the genus *Rhodococcus* were significantly decreased in the blood from the patients with pancreatic cancer as compared to the blood from the normal individuals (see Table 4 and FIG. 3).

**[Table 4]**

| Blood | Control | | Pancreatic cancer | | t-test | |
|---|---|---|---|---|---|---|
| Taxon | Mean | SD | Mean | SD | p-value | Ratio |
| *Rodocococcus* | 0.0078 | 0.0328 | 0.0012 | 0.0059 | 0.002 | 0.16 |

### Example 5. Metagenomic analysis of bacteria-derived vesicles in blood of patient with bile duct cancer

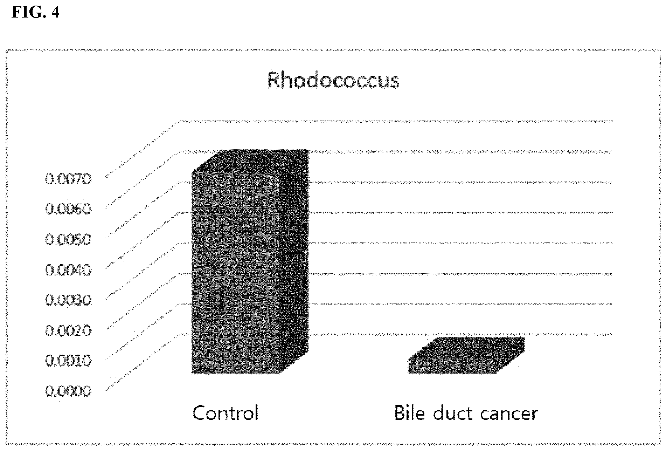

After a metagenomic analysis was performed using the method of Example 2 on the blood from 79 patients with bile duct cancer, and 159 normal individuals who were matched in age and sex by extracting genes from vesicles present in the blood, the distribution of vesicles derived from bacteria of the genus *Rhodococcus* was evaluated. As a result, it was confirmed that vesicles derived from bacteria of the genus *Rhodococcus* were significantly decreased in the blood from the patients with bile duct cancer as compared to the blood from the normal individuals (see Table 5 and FIG. 4).

**[Table 5]**

| Blood | Control | | Bile duct cancer | | t-test | |
|---|---|---|---|---|---|---|
| Taxon | Mean | SD | Mean | SD | p-value | Ratio |
| *Rodocococcus* | 0.0066 | 0.0321 | 0.0005 | 0.0016 | 0.002 | 0.07 |

### Example 6. Metagenomic analysis of bacteria-derived vesicles in blood of patient with breast cancer

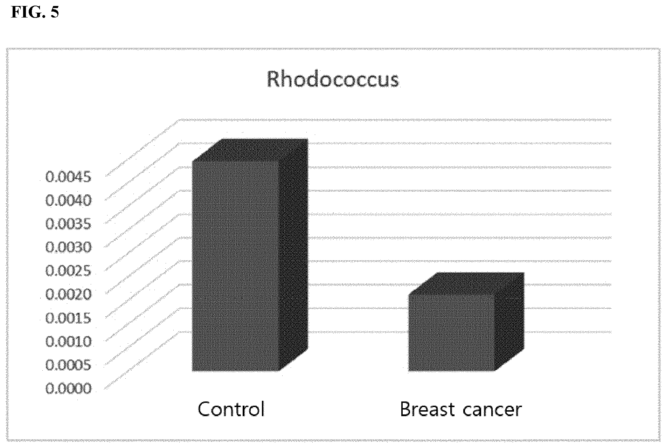

After a metagenomic analysis was performed using the method of Example 2 on the blood from 107 patients with breast cancer, and 129 normal individuals who were matched in age and sex by extracting genes from vesicles present in the blood, the distribution of vesicles derived from bacteria of the genus *Rhodococcus* was evaluated. As a result, it was confirmed that vesicles derived from bacteria of the genus *Rhodococcus* were significantly decreased in the blood from the patients with breast cancer as compared to the blood from the normal individuals (see Table 6 and FIG. 5).

**[Table 6]**

| Blood | Control | | Breast cancer | | t-test | |
|---|---|---|---|---|---|---|
| Taxon | Mean | SD | Mean | SD | p-value | Ratio |
| *Rodocococcus* | 0.0045 | 0.0089 | 0.0016 | 0.0030 | 0.0001 | 0.36 |

### Example 7. Metagenomic analysis of bacteria-derived vesicles in blood of patient with bladder cancer

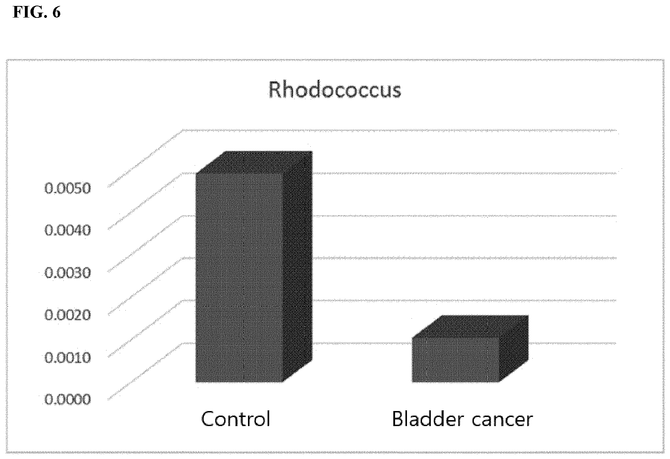

After a metagenomic analysis was performed using the method of Example 2 on the blood from 91 patients with bladder cancer, and 176 normal individuals who were matched in age and sex by extracting genes from vesicles present in the blood, the distribution of vesicles derived from bacteria of the genus *Rhodococcus* was evaluated. As a result, it was confirmed that vesicles derived from bacteria of the genus *Rhodococcus* were significantly decreased in the blood from the patients with bladder cancer as compared to the blood from the normal individuals (see Table 7 and FIG. 6).

**[Table 7]**

| Blood | Control | | Bladder cancer | | t-test | |
|---|---|---|---|---|---|---|
| Taxon | Mean | SD | Mean | SD | p-value | Ratio |
| *Rodocococcus* | 0.0049 | 0.0105 | 0.00019 | 0.0030 | 0.0001 | 0.36 |

### Example 8. Metagenomic analysis of bacteria-derived vesicles in blood of patient with lymphoma

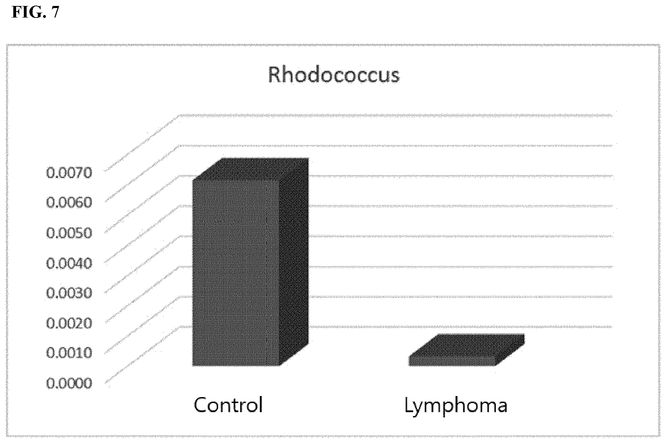

After a metagenomic analysis was performed using the method of Example 2 on the blood from 81 patients with lymphoma, and 86 normal individuals who were matched in age and sex by extracting genes from vesicles present in the blood, the distribution of vesicles derived from bacteria of the genus *Rhodococcus* was evaluated. As a result, it was confirmed that vesicles derived from bacteria of the genus *Rhodococcus* were significantly decreased in the blood from the patients with lymphoma as compared to the blood from the normal individuals (see Table 8 and FIG. 7).

**[Table 8]**

| Blood | Control | | Lymphoma | | t-test | |
|---|---|---|---|---|---|---|
| Taxon | Mean | SD | Mean | SD | p-value | Ratio |
| *Rodocococcus* | 0.0061 | 0.0200 | 0.0003 | 0.0011 | 0.04 | 0.05 |

### Example 9. Metagenomic analysis of bacteria-derived vesicles in blood of patient with diabetes

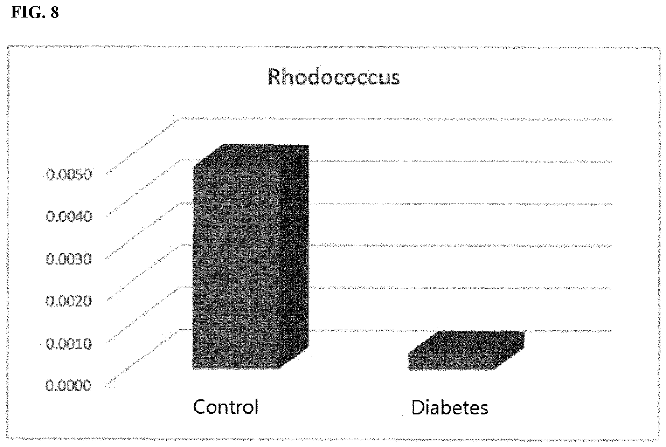

After a metagenomic analysis was performed using the method of Example 2 on the blood from 114 patients with diabetes, and 132 normal individuals who were matched in age and sex by extracting genes from vesicles present in the blood, the distribution of vesicles derived from bacteria of the genus *Rhodococcus* was evaluated. As a result, it was confirmed that vesicles derived from bacteria of the genus *Rhodococcus* were significantly decreased in the blood from the patients with diabetes as compared to the blood from the normal individuals (see Table 9 and FIG. 8).

**[Table 9]**

| Blood | Control | | Diabetes | | t-test | |
|---|---|---|---|---|---|---|
| Taxon | Mean | SD | Mean | SD | p-value | Ratio |
| *Rodocococcus* | 0.0048 | 0.0114 | 0.0004 | 0.0004 | 0.00004 | 0.07 |

### Example 10. Metagenomic analysis of bacteria-derived vesicles in blood of patient with stroke

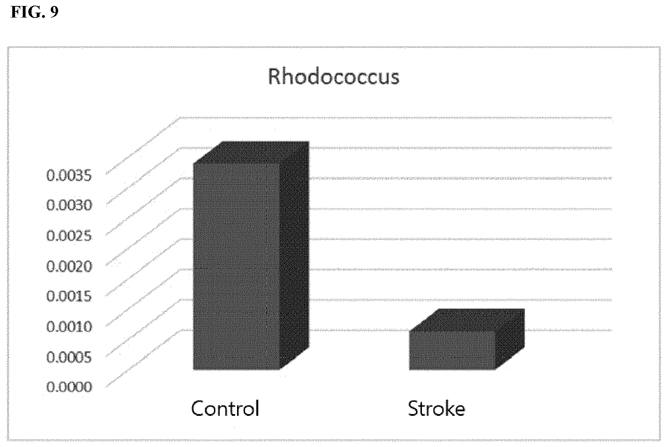

After a metagenomic analysis was performed using the method of Example 2 on the blood from 124 patients with stroke, and 142 normal individuals who were matched in age and sex by extracting genes from vesicles present in the blood, the distribution of vesicles derived from bacteria of the genus *Rhodococcus* was evaluated. As a result, it was confirmed that vesicles derived from bacteria of the genus *Rhodococcus* were significantly decreased in the blood from the patients with stroke as compared to the blood from the normal individuals (see Table 10 and FIG. 9).

**[Table 10]**

| Blood | Control | | Stroke | | t-test | |
|---|---|---|---|---|---|---|
| Taxon | Mean | SD | Mean | SD | p-value | Ratio |
| *Rodocococcus* | 0.0034 | 0.0119 | 0.0006 | 0.0025 | 0.01 | 0.19 |

### Example 11. Metagenomic analysis of bacteria-derived vesicles in blood of patient with myocardial infarction

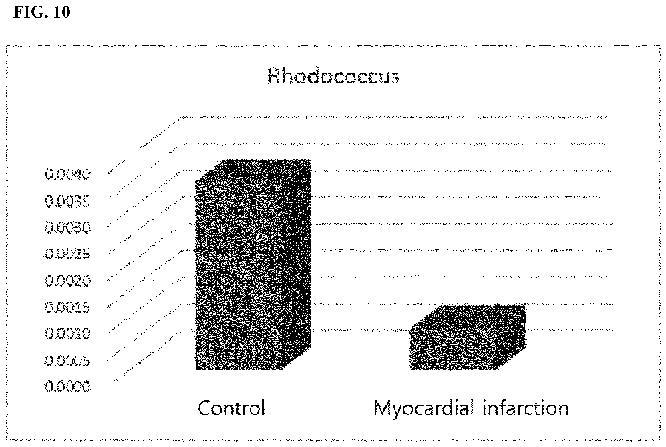

After a metagenomic analysis was performed using the method of Example 2 on the blood from 57 patients with myocardial infarction, and 113 normal individuals who were matched in age and sex by extracting genes from vesicles present in the blood, the distribution of vesicles derived from bacteria of the genus *Rhodococcus* was evaluated. As a result, it was confirmed that vesicles derived from bacteria of the genus *Rhodococcus* were significantly decreased in the blood from the patients with myocardial infarction as compared to the blood from the normal individuals (see Table 11 and FIG. 10).

**[Table 11]**

| Blood | Control | | Myocardial infarction | | t-test | |
|---|---|---|---|---|---|---|
| Taxon | Mean | SD | Mean | SD | p-value | Ratio |
| *Rodocococcus* | 0.0035 | 0.0108 | 0.0008 | 0.0023 | 0.003 | 0.22 |

### Example 12. Metagenomic analysis of bacteria-derived vesicles in blood of patient with asthma

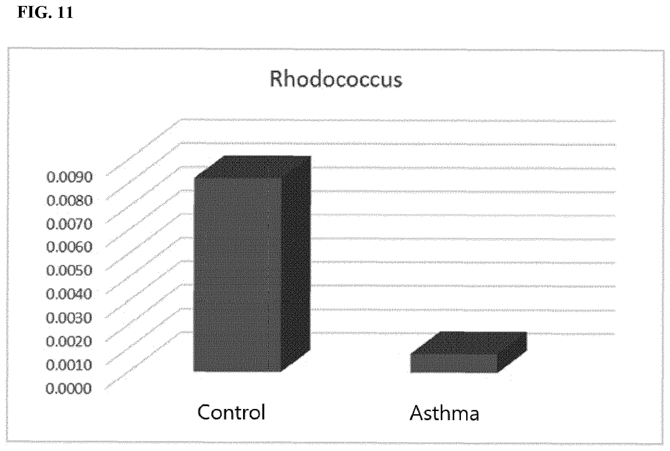

After a metagenomic analysis was performed using the method of Example 2 on the blood from 135 patients with asthma, and 164 normal individuals who were matched in age and sex by extracting genes from vesicles present in the blood, the distribution of vesicles derived from bacteria of the genus *Rhodococcus* was evaluated. As a result, it was confirmed that vesicles derived from bacteria of the genus *Rhodococcus* were significantly decreased in the blood from the patients with asthma as compared to the blood from the normal individuals (see Table 12 and FIG. 11).

**[Table 12]**

| Blood | Control | | Asthma | | t-test | |
|---|---|---|---|---|---|---|
| Taxon | Mean | SD | Mean | SD | p-value | Ratio |
| *Rodocococcus* | 0.0082 | 0.0008 | 0.0008 | 0.0019 | 0.00001 | 0.09 |

### Example 13. Metagenomic analysis of bacteria-derived vesicles in blood of patient with COPD

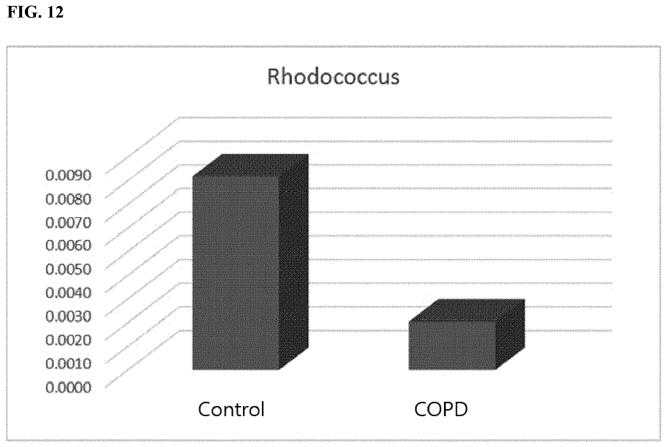

After a metagenomic analysis was performed using the method of Example 2 on the blood from 205 patients with COPD, and 231 normal individuals who were matched in age and sex by extracting genes from vesicles present in the blood, the distribution of vesicles derived from bacteria of the genus *Rhodococcus* was evaluated. As a result, it was confirmed that vesicles derived from bacteria of the genus *Rhodococcus* were significantly decreased in the blood from the patients with COPD as compared to the blood from the normal individuals (see Table 13 and FIG. 12).

**[Table 13]**

| Blood | Control | | COPD | | t-test | |
|---|---|---|---|---|---|---|
| Taxon | Mean | SD | Mean | SD | p-value | Ratio |
| *Rodocococcus* | 0.0082 | 0.0355 | 0.0020 | 0.0028 | 0.0003 | 0.25 |

### Example 14. Metagenomic analysis of bacteria-derived vesicles in blood of patient with dementia

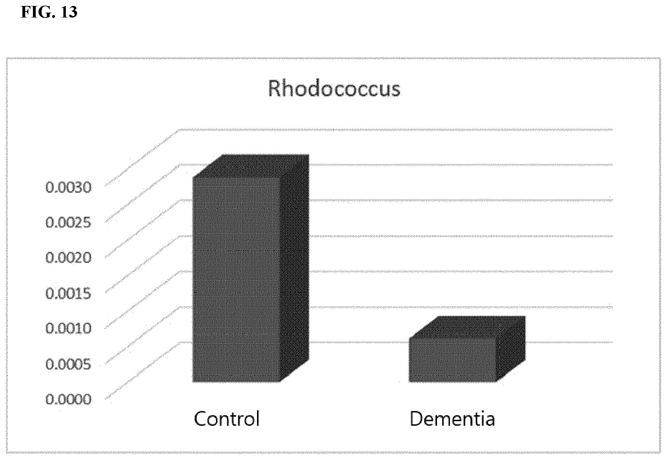

After a metagenomic analysis was performed using the method of Example 2 on the blood from 72 patients with dementia, and 93 normal individuals who were matched in age and sex by extracting genes from vesicles present in the blood, the distribution of vesicles derived from bacteria of the genus *Rhodococcus* was evaluated. As a result, it was confirmed that vesicles derived from bacteria of the genus *Rhodococcus* were significantly decreased in the blood from the patients with dementia as compared to the blood from the normal individuals (see Table 14 and FIG. 13).

**[Table 14]**

| Blood | Control | | Dementia | | t-test | |
|---|---|---|---|---|---|---|
| Taxon | Mean | SD | Mean | SD | p-value | Ratio |
| *Rodocococcus* | 0.0029 | 0.0034 | 0.0006 | 0.0011 | 0.00001 | 0.21 |

### Example 15. Isolation of Vesicles derived from Rhodococcus equi

Based on the result of the above examples, the *Rhodococcus equi* strain belonging to the genus *Rhodococcus* was cultured and then its vesicles were isolated therefrom. The *Rhodococcus equi* strain was cultured in a brain heart infusion (BHI) medium in a 37 °C anaerobic chamber to an absorbance (OD600) of 1.0 to 1.5, and then sub-cultured. Afterward, the medium supernatant not containing the strain was collected and centrifuged at 10,000 g and 4 °C for 15 minutes, filtered through a 0.45-µm filter, and the filtered supernatant was concentrated to a 200 mL volume through ultrafiltration using a QuixStand benchtop system (GE Healthcare, UK) with a 100 kDa hollow filter membrane. Afterward, the concentrated supernatant was filtered again through a 0.22-µm filter, the filtered supernatant was ultracentrifuged at 150,000 g and 4 °C for 3 hours, the pellet was suspended with Dulbecco's Phosphate Buffered Saline (DPBS). Subsequently, density-gradient centrifugation was performed using 10 %, 40 %, and 50 % OptiPrep solutions (Axis-Shield PoC AS, Norway), and to prepare a low-density solution, the OptiPrep solution was diluted with HEPES-buffered saline (20 mM HEPES, 150 mM NaCl, pH 7.4). Centrifugation was performed at 200,000 g and 4 °C for 2 hours, and each solution fractionated into the same volume of 1 mL from the upper layer was additionally ultracentrifuged at 150,000 g and 4 °C for 3 hours. Afterward, a protein was quantified using a bicinchoninic acid (BCA) assay, and an experiment was performed on the obtained vesicles.

### Example 16. Anti-inflammatory Effects of Vesicles derived from Rhodococcus equi

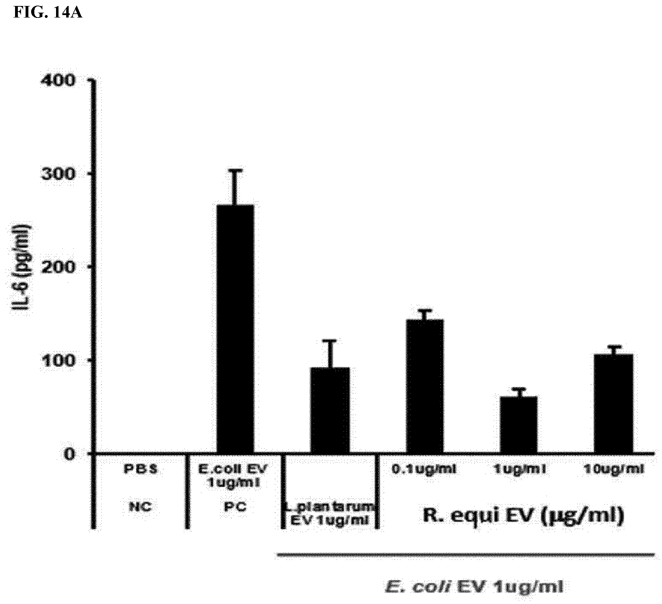

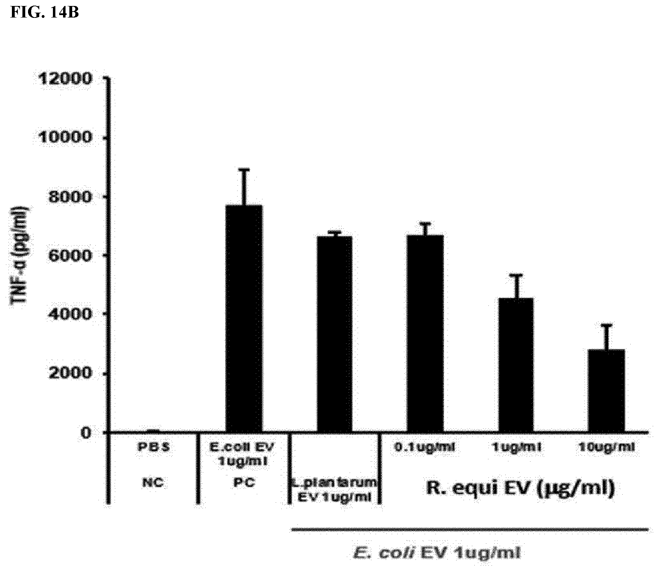

To examine the effect of *Rhodococcus* equi-derived vesicles on the secretion of inflammation mediators in inflammatory cells, Raw 264.7 cells, which is a mouse macrophage cell line, were treated with the *Rhodococcus* equi-derived vesicles at various concentrations (0.1, 1 and 10 µg/mL), and then treated with *E. coli* EVs, which are pathogenic vesicles of an inflammatory disease, followed by measuring secretion amounts of the inflammation mediators (IL-6 and TNF-α). More specifically, the Raw 264.7 cells were seeded in a 24-well cell culture plate at 1 × 10⁵ cells/well, and cultured in complete DMEM for 24 hours. Afterward, a culture supernatant was collected in a 1.5 mL tube and centrifuged at 3000 g for 5 minutes, and then the resulting supernatant was stored at 4 °C and subjected to ELISA analysis. As a result, when *Rhodococcus* equi-derived vesicles were pretreated, it was confirmed that the secretion of the IL-6 and TNF-α by *E. coli* EVs was significantly suppressed (see FIG. 14A and 14B). This result shows that inflammatory responses induced by pathogenic vesicles such as E. coli-derived vesicles can be effectively inhibited by the *Rhodococcus equi-derived* vesicles.

### Example 17. Nerve cell protective effect of Rhodococcus equi-derived vesicles

A brain-derived neurotrophic factor (BDNF) is a major mediator protecting nerve cells from nerve cell damage, and its expression is decreased in dementia, depression, Alzheimer's disease, autism, and the like. In this example, to evaluate the therapeutic effect of *Rhodococcus* equi-derived vesicles against a cranial nerve disease, the nerve cell protective effect was evaluated by treating the nerve cells with a stress hormone. That is, nerve cells (hippocampal neuronal cell line, HT22 cells) were cultured with an adrenocortical hormone (GC: corticosterone 400 ng/ml) or *Rhodococcus* equi-derived vesicles (EV, 20 µg/mL) for 24 hours in vitro, and the BDNF expression was evaluated by PCR.

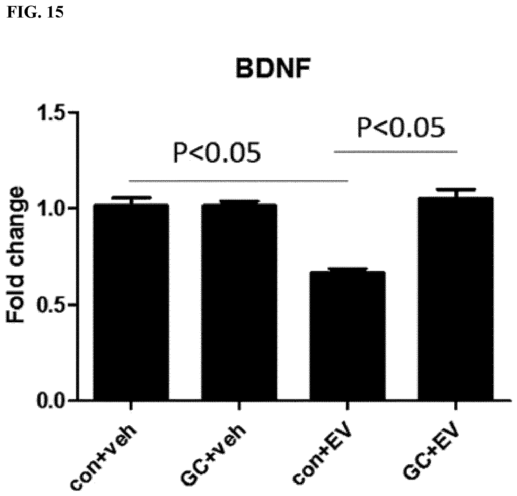

As a result, it was confirmed that the BDNF expression inhibited by adrenocortical hormone treatment is significantly recovered by the treatment of *Rhodococcus equi-derived* vesicles (see FIG. 15). This means that the *Rhodococcus* equi-derived vesicles can effectively inhibit a cranial nerve disease caused by a factor causing cranial nerve cell damage such as stress.

The above-described description of the present invention is provided for illustrative purposes, and those of ordinary skill in the art to which the present invention pertains will understand that the present invention can be easily modified into other specific forms without changing the technical spirit or essential features of the present invention. Therefore, it should be understood that the above-described Examples are illustrative only in all aspects and are not restrictive.

### [Industrial Applicability]

Vesicles derived from bacteria of the genus *Rhodococcus* according to the present invention are expected to be effectively used in a method of diagnosing lung cancer, pancreatic cancer, bile duct cancer, breast cancer, bladder cancer, lymphoma, diabetes, stroke, myocardial infarction, asthma, COPD, or dementia; and a food, inhalant, cosmetic or pharmaceutical composition for preventing, alleviating or treating a malignant disease, diabetes, stroke, a cardiovascular disease, an inflammatory pulmonary disease, or a cranial nerve disease.

## Claims

1. A method of providing information for diagnosing lung cancer, pancreatic cancer, bile duct cancer, breast cancer, bladder cancer, lymphoma, diabetes, stroke, myocardial infarction, asthma, chronic obstructive pulmonary disease (COPD), or dementia, the method comprising the following steps:
(a) extracting DNAs from vesicles isolated from samples of a normal individual and a subj ect;
(b) performing PCR (Polymerase Chain Reaction) on the extracted DNA using a pair of primers prepared based on a gene sequence present in 16S rDNA to obtain each PCR product; and
(c) classifying a case in which a content of vesicles derived from bacteria of the genus *Rhodococcus* is lower than that of the normal individual sample, as lung cancer, pancreatic cancer, bile duct cancer, breast cancer, bladder cancer, lymphoma, diabetes, stroke, myocardial infarction, asthma, chronic obstructive pulmonary disease (COPD), or dementia, through quantitative analysis of the PCR product.

2. The method of claim 1, wherein the sample in Step (a) is blood or urine.

3. A pharmaceutical composition for preventing or treating one or more diseases selected from the group consisting of a malignant disease, diabetes, stroke, a cardiovascular disease, an inflammatory pulmonary disease, and a cranial nerve disease, comprising vesicles derived from bacteria of the genus *Rhodococcus* as an active ingredient.

4. The pharmaceutical composition of claim 3, wherein the malignant disease is one or more selected from the group consisting of lung cancer, pancreatic cancer, bile duct cancer, breast cancer, bladder cancer, and lymphoma.

5. The pharmaceutical composition of claim 3, wherein the cardiovascular disease is one or more selected from the group consisting of myocardial infarction, hypertension, ischemic heart disease, coronary artery disease, angina, atherosclerosis (arteriosclerosis), and arrhythmia.

6. The pharmaceutical composition of claim 3, wherein the inflammatory pulmonary disease is one or more selected from the group consisting of asthma, chronic obstructive pulmonary disease (COPD), acute lung injury, empyema, lung abscesses, pneumonia, pulmonary tuberculosis, and bronchitis.

7. The pharmaceutical composition of claim 3, wherein the cranial nerve disease is one or more selected from the group consisting of depression, obsessive-compulsive disorder, schizophrenia, dementia, Alzheimer's disease, epilepsy, autism, and Parkinson's disease.

8. The pharmaceutical composition of claim 3, wherein the vesicles have an average diameter of 10 to 200 nm.

9. The pharmaceutical composition of claim 3, wherein the vesicles are secreted naturally or artificially from bacteria of the genus *Rhodococcus.*

10. The pharmaceutical composition of claim 3, wherein the vesicles derived from bacteria of the genus *Rhodococcus* are secreted from *Rhodococcus equi.*

11. A food composition for preventing or alleviating one or more diseases selected from the group consisting of a malignant disease, diabetes, stroke, a cardiovascular disease, an inflammatory pulmonary disease, and a cranial nerve disease, comprising vesicles derived from bacteria of the genus *Rhodococcus* as an active ingredient.

12. The food composition of claim 11, wherein the vesicles have an average diameter of 10 to 200 nm.

13. The food composition of claim 11, wherein the vesicles are secreted naturally or artificially from bacteria of the genus *Rhodococcus.*

14. The food composition of claim 11, wherein the vesicles derived from bacteria of the genus *Rhodococcus* are secreted from *Rhodococcus equi.*

15. An inhalant composition for preventing or treating one or more diseases selected from the group consisting of a malignant disease, diabetes, stroke, a cardiovascular disease, an inflammatory pulmonary disease, and a cranial nerve disease, comprising vesicles derived from bacteria of the genus *Rhodococcus* as an active ingredient.

16. A cosmetic composition for preventing or alleviating an inflammatory skin disease, comprising vesicles derived from bacteria of the genus *Rhodococcus* as an active ingredient.

17. The cosmetic composition of claim 16, wherein the inflammatory skin disease is one or more selected from the group consisting of atopic dermatitis, acne, hair loss, and psoriasis.

18. A method of diagnosing lung cancer, pancreatic cancer, bile duct cancer, breast cancer, bladder cancer, lymphoma, diabetes, stroke, myocardial infarction, asthma, chronic obstructive pulmonary disease (COPD), or dementia, the method comprising the following steps:
(a) extracting DNAs from vesicles isolated from samples of a normal individual and a subject;
(b) performing PCR on the extracted DNA using a pair of primers prepared based on a gene sequence present in 16S rDNA to obtain each PCR product; and
(c) determining a case in which a content of vesicles derived from bacteria of the genus *Rhodococcus* is lower than that of the normal individual sample, as lung cancer, pancreatic cancer, bile duct cancer, breast cancer, bladder cancer, lymphoma, diabetes, stroke, myocardial infarction, asthma, chronic obstructive pulmonary disease (COPD), or dementia, through quantitative analysis of the PCR product.

19. The method of claim 18, wherein the sample in Step (a) is blood or urine.

20. A method of preventing or treating one or more diseases selected from the group consisting of a malignant disease, diabetes, stroke, a cardiovascular disease, an inflammatory pulmonary disease, and a cranial nerve disease, the method comprising a step of administering a pharmaceutical composition comprising vesicles derived from bacteria of the genus *Rhodococcus* as an active ingredient to a subject.

21. A use of a pharmaceutical composition comprising vesicles derived from bacteria of the genus *Rhodococcus* as an active ingredient for preventing or treating one or more diseases selected from the group consisting of a malignant disease, diabetes, stroke, a cardiovascular disease, an inflammatory pulmonary disease, and a cranial nerve disease.

22. A use of vesicles derived from bacteria of the genus *Rhodococcus* for producing a drug used in treating one or more diseases selected from the group consisting of a malignant disease, diabetes, stroke, a cardiovascular disease, an inflammatory pulmonary disease, and a cranial nerve disease.


<110>     MD Healthcare Inc.

<120>     Nanovesicles derived from Rhodococcus bacteria and Use thereof

<130>     MPCT20-002

<150>     KR 10-2019-0002609
<151>     2019-01-09

<150>     KR 10-2020-0002274
<151>     2020-01-07

<160>     2

<170>     KoPatentIn 3.0

<210>     1
<211>     50
<212>     DNA
<213>     Artificial Sequence

<220>
<223>     16S_V3_F


<220>
<221>     misc_feature
<222>     (42)
<223>     n = a or g or c or t/u


<400>     1
tcgtcggcag cgtcagatgt gtataagaga cagcctacgg gnggcwgcag                50


<210>     2
<211>     55
<212>     DNA
<213>     Artificial Sequence

<220>
<223>     16S_V4_R


<400>     2
gtctcgtggg ctcggagatg tgtataagag acaggactac hvgggtatct aatcc          55